# FASCICULE DE BREVET EUROPEEN

(11) **EP 2 542 662 B1**
(45) Date de publication et mention de la délivrance du brevet: **06.12.2017**
(21) Numéro de dépôt: 11705631.7
(22) Date de dépôt: 02.03.2011
(51) Int. Cl.: B01L 3/00, B01L 9/00, C12M 1/32, C12M 1/34, C12M 3/00

(54) **BOITE MULTI-REACTEURS POUR CULTURE CELLULAIRE DYNAMIQUE**
MULTIREAKTOREINHEIT FÜR DYNAMISCHE ZELLKULTUR
MULTI-REACTOR UNIT FOR DYNAMIC CELL CULTURE

(30) Priorité: 02.03.2010 FR 1051482; 02.03.2010 FR 1051480
(43) Date de publication de la demande: 09.01.2013
(73) Titulaire: Université Technologie de Compiègne-UTC, 60203 Compiegne cedex (FR); Centre National de la Recherche Scientifique (C.N.R.S.), 75016 Paris (FR)
(72) Inventeur: LEGALLAIS, Cécile, F-60340 Villers Sous Saint Leu (FR); BAUDOIN, Régis, F-93360 Neuilly Plaisance (FR); LECLERC, Eric, F-60280 Margny Les Compiegne (FR); PROT, Jean-Matthieu, Ithaca, NY 14850 (US); PAULLIER, Patrick, 60150 Thourotte (FR)
(74) Mandataire: Regimbeau
(86) Numéro de dépôt international: PCT/EP2011/053128
(87) Numéro de publication internationale: WO 2011/107519

(56) Documents cités:
- WO-A1-2006/097749
- WO-A2-2009/024595
- US-A1- 2005 260 745
- US-A1- 2007 243 523
- BAUDOIN RÉGIS: "Développement et caractérisation d'une puce à cellules pour le calibrage d'agents toxiques", THESIS PRÉSENTÉE POUR L'OBTENTION DU GRADE DE DOCTEUR DE L'UTC, XX, XX , 27 novembre 2008 (2008-11-27), pages 1-61, XP002576413, Extrait de l'Internet: URL:http://tel.archives-ouvertes.fr/docs/0 0/34/23/42/PDF/Rapport_THESE_Ve rsion_finale.pdf [extrait le 2010-04-01]

## Description

### DOMAINE TECHNIQUE GENERAL

La présente invention concerne la culture in vitro de cellules

Plus précisément, elle concerne une boîte multi-bioréacteurs pour culture cellulaire dynamique en milieu nutritif.

### ETAT DE L'ART

Les cultures de cellules sont aujourd'hui utilisées comme outil d'évaluation toxicologique de substances.

On dispose également de bioréacteurs reproduisant un environnement favorable au développement et à l'organisation de cellules, proche de celui d'un tissu ou d'un organe animal ou humain. Grâce à eux, on peut prévoir la réaction d'un organe vis-à-vis d'une substance comme un xénobiotique, un cosmétique, un médicament ou plus généralement tout principe actif, et développer des modèles in vitro pertinents.

Ces modèles sont de plus en plus souvent utilisés dans la recherche pharmaceutique car ils représentent une alternative sérieuse aux modèles in vivo, c'est à dire l'expérimentation animale, contre laquelle des pressions à la fois économiques et éthiques apparaissent au niveau international.

La culture de cellules représente également la base de la création d'organes artificiels capables de se substituer aux organes défaillants ou absents, un des enjeux de l'ingénierie tissulaire.

Les bioréacteurs comprennent aux deux extrémités d'une chambre microstructurée (une chambre de culture comportant une paroi supérieure et une paroi inférieure présentant des microstructures) un point d'entrée de fluide et un point de sortie de fluide pour permettre le passage d'un fluide nutritif nécessaire au développement des cellules. Le fluide est donc en circulation, mu par exemple par une pompe, c'est ce que l'on appelle une culture cellulaire dynamique. Un tel dispositif a été décrit dans la demande de brevet FR0954288 ou dans le document Baudouin Régis, 27 novembre 2008, pages 1-61, XP002576413.

Ce bioréacteur précurseur offre d'excellents résultats et permet un développement optimal des cellules, mais ne peut tout seul suffire à un usage industriel. Il serait souhaitable de pouvoir augmenter autant que possible la surface de culture des cellules.

Une solution pour cela est d'utiliser des multi-bioréacteurs, c'est-à-dire des bioréacteurs parallélisés. Ces dispositifs devraient en outre présenter un encombrement réduit. Divers systèmes de culture cellulaire intégrée ont été proposés à cet effet.

Le système Hµrel®, décrit par les brevets US5612188 et US7288405, est constitué de quatre doubles bioréacteurs disposés en parallèle et reliés à une pompe péristaltique. Un réservoir est également inséré dans le circuit. Celui-ci sert de réserve de milieu nutritif, mais aussi d'espace de prélèvement d'échantillons.

En effet des prélèvements s'effectuent tout au long de la culture cellulaire pour surveiller son bon déroulement, et lors de l'utilisation des cellules à des fins d'étude toxicologique : après exposition à une substance à tester, une série de prélèvements réguliers permet par exemple de déterminer la réponse métabolique des cellules à cette substance, de mesurer la clairance (c'est-à-dire la capacité de l'organe à éliminer la substance dans un volume donné), ou encore d'observer les cinématiques de réaction des cellules.

Or la procédure d'échantillonnage du système Hµrel® (dans un réservoir) est longue et complexe, puisqu'il est nécessaire d'arrêter la perfusion de fluide pour pouvoir prélever un échantillon de milieu dans le réservoir. De plus ce prélèvement s'effectue loin des bioréacteurs, ce qui fait qu'il y a un risque que des marqueurs métaboliques recherchés via les prélèvements voient leur concentration chuter entre le bioréacteur et le réservoir, à cause de phénomènes d'adsorption sur la surface des tuyaux.

On notera de plus que l'introduction des bioréacteurs dans le circuit de perfusion nécessite une opération longue et délicate dans des conditions stériles pour éviter la formation de bulles potentiellement très néfastes pour les cellules en développement. L'adjonction d'un piège à bulles dans le circuit est par ailleurs nécessaire.

En outre, ces bioréacteurs sont entre deux plaques de plusieurs centimètres d'épaisseur qui ne dispose pas de connectiques. Le branchement se fait donc via des embouts placés dans des trous des plaques.

Ce système assure une étanchéité, mais est problématique lors de l'ensemencement en cellules. En effet les souches de cellules humaines sont extrêmement chères, et doivent être mises en place précautionneusement. La structure de ce dispositif impose d'ensemencer deux bioréacteurs consécutifs avec les mêmes cellules, d'où des redondances.

Par ailleurs, en plus de complexifier le réseau de perfusion puisqu'il en faut un de chaque par bioréacteur parallélisé, les éventuels réservoirs et pièges à bulles rallongent les circuits, et augmentent d'autant la surface interne des tuyaux. Or cette surface est le siège d'une adsorption des substances chimiques en solution. Cette adsorption fausse les tests de toxicologie, car un xénobiotique injecté en début de circuit verrait sa concentration chuter, voire tomber à zéro avant même de rencontrer les cellules en culture en fin de circuit. Le risque de faux négatif n'est pas négligeable.

Les systèmes Bioflux™ 200 et Bioflux™ 1000, décrits par la demande de brevet US2007/0243523, proposent une boîte étanche utilisant une plaque multi-puits du commerce adaptée. Une plaque multi-puits est une plaque standardisée comportant de nombreux puits ayant le rôle de petites éprouvettes. Il s'agit d'un outil couramment utilisé dans les laboratoires, disponible en de très nombreux modèles. Dans cette plaque adaptée, des microcanaux reliant deux puits servent de support à la croissance des cellules. Le prélèvement se fait ainsi dans le second puits, il s'agit donc d'un prélèvement in situ au plus près de la zone de culture. Cependant les problèmes de complexité de prélèvement sont encore pires que dans le système Hµrel®, car il faut décapoter entièrement la plaque multi-puits, et donc arrêter le fonctionnement de la totalité des réacteurs parallélisés.

Par ailleurs, le système Bioflux™ est bien moins favorable à la culture des cellules : il ne permet pas la culture cellulaire dynamique recirculante, car la perfusion du milieu de culture se fait par l'application d'une surpression. Il n'y a pas de microstructures, mais un simple canal entre deux puits. L'adhérence et la surface efficace sont très faibles et le rendement chute. Il faut injecter en aveugle une énorme quantité de cellules pour ensemencer un tel dispositif. Sachant qu'une dose pour ensemencer un seul microbioréacteur, de l'ordre du million de cellules, coûte plusieurs centaines d'euros, il est essentiel d'améliorer les procédés d'ensemencement cellulaire pour pouvoir en faire un outil rentable.

Des moyens de prélèvement issus d'autres domaines technologiques ont également été proposés, mais aucun ne donne pleinement satisfaction.

Un premier système possible est d'utiliser une voie de dérivation, au bout de laquelle on va pouvoir prélever. Toutefois le fluide présent dans cette voie de dérivation stagne, et va la polluer par des dépôts. Il est nécessaire de la nettoyer avant chaque prélèvement si l'on veut une mesure fiable, et pour ce on retrouve les problèmes de l'art antérieur (arrêt et démontage du circuit), à moins de mettre en place un deuxième circuit complet pour effectuer le nettoyage, avec toutes les difficultés et le coût que cela représente.

Un autre système envisagé utilise un septum, c'est-à-dire un petit bouchon en caoutchouc, comprimé, fermant un orifice dans le circuit à prélever. Les septums sont notamment utilisés en médecine (sur la tête des cathéters par exemple). On prélève en traversant le bouchon avec l'aiguille d'une seringue, le trou créé se refermant naturellement par déformation du matériau lorsque l'on retire l'aiguille. Néanmoins, un septum est à changer après une dizaine de prélèvements (arrêt et démontage du circuit à nouveau requis). De plus l'utilisation d'une seringue est adaptée pour prélever des volumes d'au moins plusieurs millilitres, ce qui s'avère incompatible avec les circuits classiques de culture cellulaire, dont le volume total est généralement de 2-3mL. Les systèmes cités précédemment utilisent des micropipettes autorisant des prélèvements de seulement quelques dizaines de microlitres.

Outre ce qui a été mentionné ci-dessus à propos des systèmes connus, qui peuvent chacun être associés à des inconvénients ou limitations particuliers, on remarque qu'aucun des systèmes connus n'offre la possibilité d'observer facilement les cellules en culture pendant le fonctionnement. En effet, il serait intéressant de pouvoir suivre visuellement la prolifération des cellules au cours du temps. Ceci constitue l'objectif principal de l'invention.

### PRESENTATION DE L'INVENTION

La présente invention vise par ailleurs, de manière annexe mais avantageuse, à résoudre les difficultés précédemment énoncées en proposant un dispositif qui, par une structure simple, permet de paralléliser des bioréacteurs performants de culture cellulaire dynamique tout en donnant accès à un ou plusieurs des avantages suivants :
- s'affranchir de réservoirs ou de piège à bulles externes,
- réduire la longueur de circuit nécessaire
- permettre un prélèvement de milieu de culture in situ en fonctionnement.

Un autre but annexe de l'invention est de parvenir à cet objectif tout en obtenant un produit plus facile à utiliser, ne présentant plus de risque de destruction du milieu cellulaire par une bulle, et dans lequel des très petits volumes peuvent être prélevés avec grande précision en un minimum de manipulations.

Un autre but annexe encore est de permettre un ensemencement cellulaire contrôlé, rapide, et plus économique.

Un premier aspect de l'invention concerne une boîte comprenant une chambre microstructurée de culture cellulaire dynamique.

Selon un premier aspect de l'invention, la présente invention se rapporte à une boîte comprenant un microsystème et au moins une connectique d'interface du microsystème ; le microsystème comprenant une plaque inférieure portant l'empreinte d'au moins une chambre microstructurée de culture cellulaire dynamique, et une plaque supérieure, caractérisée en ce que l'au moins une chambre microstructurée est branchée fluidiquement en entrée et/ou en sortie par l'au moins une connectique insérée de façon amovible dans un trou de la plaque supérieure, la boîte comprenant en outre au moins un puits, la chambre microstructurée étant connectée à un puits via une des connectiques.

Grâce à cette structure, le microsystème peut être aisément détaché à tout moment, placé sous un microscope pour observation, puis remis en place.

Selon d'autres caractéristiques avantageuses et non limitatives :
- le fond du puits est à une altitude supérieure à tout point de la chambre ;
- la connectique est un connecteur fixé à un trou au fond du puits ;
- chaque chambre microstructurée est connectée à un puits d'entrée et un puits de sortie perfusés chacun par une connectique respectivement d'entrée et de sortie ;
- la boîte est constituée d'un boitier inférieur recouvert d'un capot étanche maintenu par des moyens de serrage ;
- le boitier inférieur comprend le microsystème et une plaque multi-puits, les puits étant des puits de ladite plaque multi-puits ;
- la plaque multi-puits est une plaque jetable en polystyrène ;
- la plaque multi-puits est une plaque autoclavable en polycarbonate ;
- le capot comprend des connectiques mâle perfusant les puits et des connectiques femelle d'entrée ;
- la boîte comprend un connecteur male et un connecteur femelle par puits d'entrée et un connecteur maie et deux connecteurs femelle par puits de sortie ;
- le microsystème est constitué de polydimethylsiloxane ;
- un insert à membrane est disposé dans au moins l'un des puits.

L'invention propose également, selon un deuxième aspect de l'invention, un système de culture cellulaire dynamique caractérisé en ce qu'il comprend :
- une boîte selon le premier aspect de l'invention, et
- au moins un circuit de fluide comprenant une tuyauterie de circulation munie d'un moyen de circulation et connectée à au moins une chambre microstructurée.

Selon d'autres caractéristiques avantageuses et non limitatives de ce système :
- le moyen de circulation est une pompe péristaltique multicanaux.

Grâce à la suppression des trappes à bulles et des réservoirs, il est possible de réduire la longueur de circuit de perfusion de moitié. Les résultats d'études toxicologiques sur un système de culture cellulaire selon le deuxième aspect de l'invention en seront d'autant plus fiables vis-à-vis de tests similaires effectués sur des dispositifs connus.

L'invention propose enfin, selon un troisième aspect, une méthode d'ensemencement cellulaire d'une boîte selon le premier aspect de l'invention, caractérisée en ce qu'elle comprend des étapes de :
a) Ouverture du capot ;
b) Introduction de cellules à ensemencer dans une pipette ;
c) Insertion de la pointe de la pipette dans une connectique ;
d) Injection de cellules directement dans la chambre microstructurée depuis la pipette ;
e) Fermeture étanche du capot et verrouillage par les moyens de serrage.

Selon d'autres caractéristiques avantageuses et non limitatives de cette méthode :
- la pipette est une micropipette jaugée, calibrée pour contenir une quantité prédéterminée de cellules correspondant à l'ensemencement optimal d'une chambre microstructurée ;
- les étapes b), c) et d) sont répétées autant de fois qu'il y a de chambres dans le microsystème ;
- la pipette est une pipette multicanaux à écartement adapté à la plaque multi-puits.

### PRESENTATION DES FIGURES

D'autres caractéristiques et avantages de la présente invention apparaîtront à la lecture de la description qui va suivre d'un mode de réalisation préférentiel. Cette description sera donnée en référence aux dessins annexés dans lesquels :
- La figure 1 est une vue éclatée en perspective d'une boîte selon l'invention ;
- les figures 2a, 2b et 2c sont trois coupes transversales de la boîte selon l'invention, décapotée dans la 2c. La figure 2a montre un prélèvement de fluide, la figure 2b met en évidence le chemin fluidique dans la boîte, et la figure 2c montre l'ensemencement cellulaire d'une chambre microstructurée ;
- la figure 3a est une vue en perspective du capot de la boîte selon l'invention dans lequel est montré un possible système de fermeture des ports de prélèvement ;
- la figure 3b est une vue éclatée en perspective du capot de la boîte selon l'invention ;
- la figure 4 est une vue éclatée en perspective du boitier inférieur de la boîte selon l'invention ;
- la figure 5 est une vue éclatée en perspective du microsystème de la boîte selon l'invention ;
- la figure 6 est une coupe transversale d'une autre réalisation possible du boitier inférieur de la boîte selon l'invention, dans lequel le microsystème est détachable ;
- les figures 7a-7c représentent trois types possibles d'une plaque multi-puits utilisables dans divers modes de réalisation de la boîte selon l'invention ;
- les figures 8a-8b sont des exemples d'observations au microscope de cellules en culture dans divers modes de réalisation de la boîte selon l'invention ;
- la figure 9 est un schéma d'un système de culture cellulaire dynamique selon l'autre aspect de l'invention ;
- la figure 10 est un graphique représentant les débits fluidiques obtenus lors d'expériences en fonction de la vitesse de fonctionnement de la pompe pour différents calibres de tuyaux.

### DESCRIPTION DETAILLEE

### Architecture générale

La boîte 1 conforme à l'invention vise préférentiellement la parallélisation des bioréacteurs. Elle sera ainsi désignée dans certains modes de réalisation en tant que boîte multi-réacteurs. Toutefois, l'invention n'est pas limitée à ce mode de réalisation et peut ne comprendre qu'une chambre microstructurée 15.

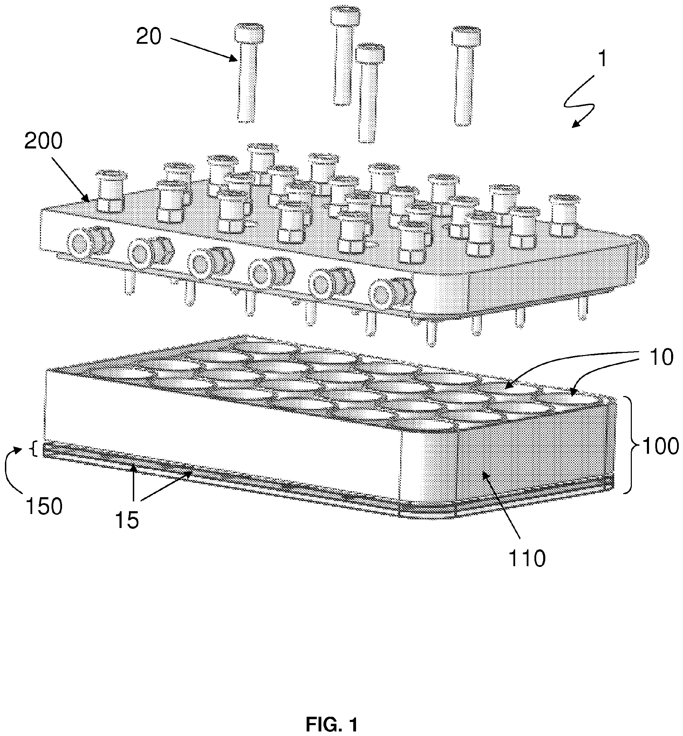

Dans le mode de réalisation préféré illustré par les figures, la boîte 1 est constituée d'un boitier inférieur 100 que vient recouvrir un capot étanche 200 maintenu par un système de serrage 20, par exemple quatre vis le traversant, comme représenté sur la figure 1.

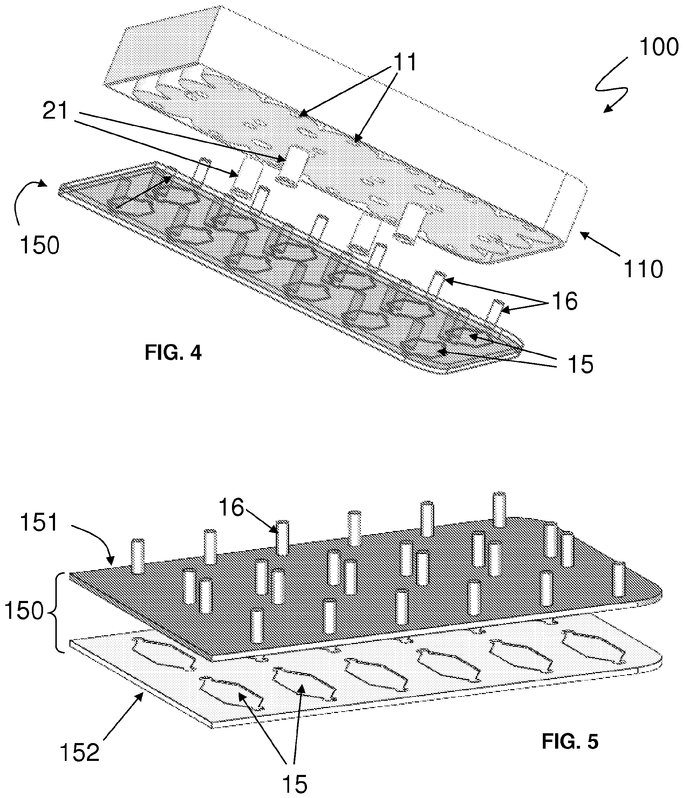

Ce boitier 100, que l'on voit mieux en détail sur la figure 4, comprend une plaque multi-puits 110, parmi lesquels on va trouver le ou les puits 10, et un microsystème 150. Des entretoises filetées 21 peuvent être disposées entre les puits 10 de façon complémentaire avec les moyens de serrage 20. Ces entretoises 21 peuvent être partie intégrante de certaines plaques multi-puits 110, ou être des tubes insérés dans des orifices percés dans le boitier, et collés.

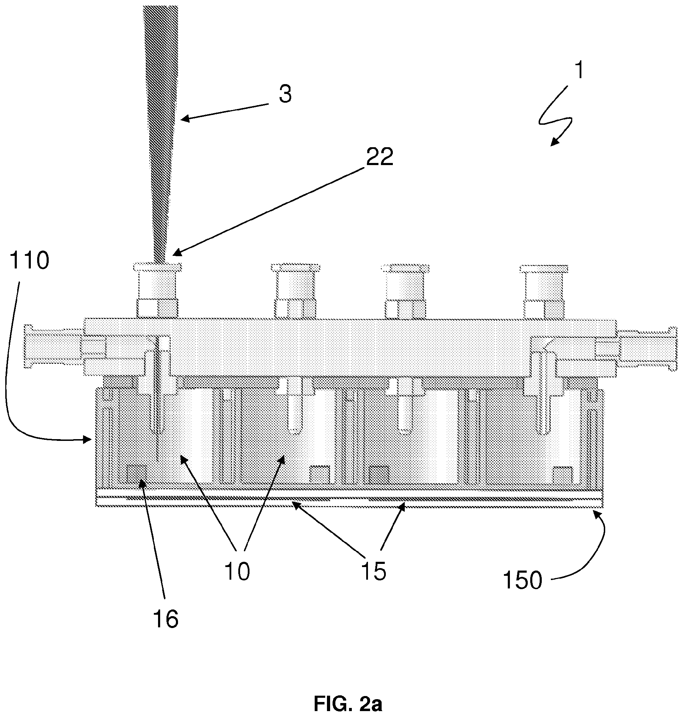

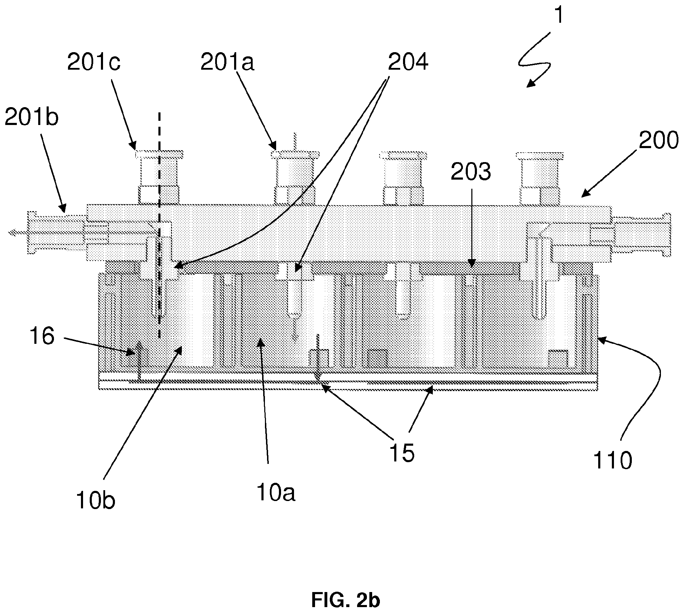

La plaque multi-puits 110 est un objet bien connu de l'homme du métier qui peut ainsi se décliner sous de nombreuses formes, et qui est parfois appelé plaque microtitre, en référence aux petits volumes calibrés des puits. Par convention, ceux-ci sont disposés selon une matrice de rapport 2 :3, et on trouve généralement des plaques de 6, 12, 24, 96 ou 384 puits dans le commerce. Avantageusement, pour des considérations liées au volume des puits, la plaque multi-puits 110 conforme à l'invention est une plaque 24 puits. Une telle boîte contient six rangées de quatre puits 10 agencés comme le montrent la figure 2a ou 2b. Dans cette architecture, les puits centraux sont des puits d'entrée 10a, et les deux puits latéraux sont des puits de sortie 10b.

Au fond des puits 10, sont percés des trous 11 par lesquels s'effectuent les communications de fluide entre les puits 10 et les chambres microstructurées 15. Chaque trou 11 permet d'ensemencer efficacement, puisque les souches de cellules injectées dans ce trou tombent dans la chambre microstructurée 15 à laquelle il est relié. Il n'y a pas de volume mort dans lequel les cellules viendraient se déposer avant d'atteindre la chambre, comme dans l'art antérieur.

### Prélèvement de fluide

La figure 2a représente la boîte dans un mode de réalisation préféré, en situation de prélèvement de fluide. La pointe d'une pipette 3 de prélèvement est insérée dans un port 22. Ce port 22 est situé à une altitude supérieure à tout point du puits 10 en dessous de lui. Par altitude supérieure, on sous-entend des conditions telles que toute goutte de fluide du puits 10 possède moins d'énergie potentielle de pesanteur qu'une goutte qui passerait le port 22. Cela est le cas en plaçant la boîte 1 selon l'invention dans une position de fonctionnement normale, c'est-à-dire avec le fond du boitier inférieur 100 posé sur une surface proche de l'horizontale.

Cette structure permet de faire des prélèvements de fluide du puits 10 sans arrêter la perfusion, contrairement à ce qui se faisait jusque là.

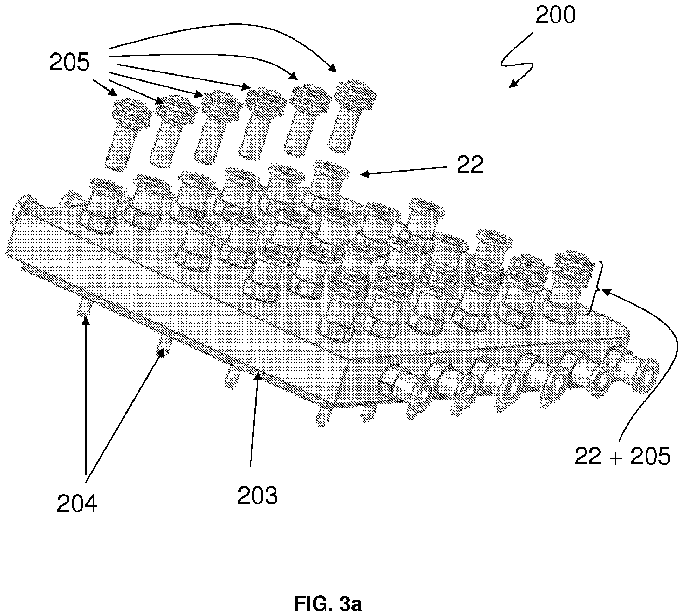

En effet le port 22 est fermé en conditions normales de fonctionnement. Pour cela il comprend un bouchon 205, ou tout autre dispositif équivalent rendant le port 22 facilement ouvrable et refermable. On peut en voir un exemple sur la figure 3a. De par la différence d'altitude entre le port 22 et le puits 10, on ne trouve pas de fluide immédiatement sous le bouchon 205, mais un peu d'air piégé. Ce qui fait que lorsqu'on ouvrira le port 22 pour faire un prélèvement, il n'y a pas de risque immédiat de débordement de fluide, ni de problème de fluide qui pourrait stagner et polluer le port 22.

Le prélèvement s'effectue ainsi en ouvrant le bouchon 205 du port 22, introduisant rapidement la pointe de la pipette 3 de prélèvement jusque dans le puits 10, aspirant un volume requis, avant de retirer la pipette 3 et refermer. Le puits 10 équipé du port 22 est un puits de sortie 10b, c'est-à-dire qu'il reçoit le fluide juste après qu'il soit passé dans la chambre microstructurée 15 à laquelle ce puits 10b est relié.

Avantageusement, le port 22 traverse le capot étanche 200 de façon à permettre le prélèvement sans nécessiter de décapoter la boîte 1, comme c'est souvent le cas pour les systèmes connus.

### Structure du capot

On rappelle que le capot étanche 200 est un élément facultatif de l'invention qui peut faire l'objet de nombreux modes de réalisation.

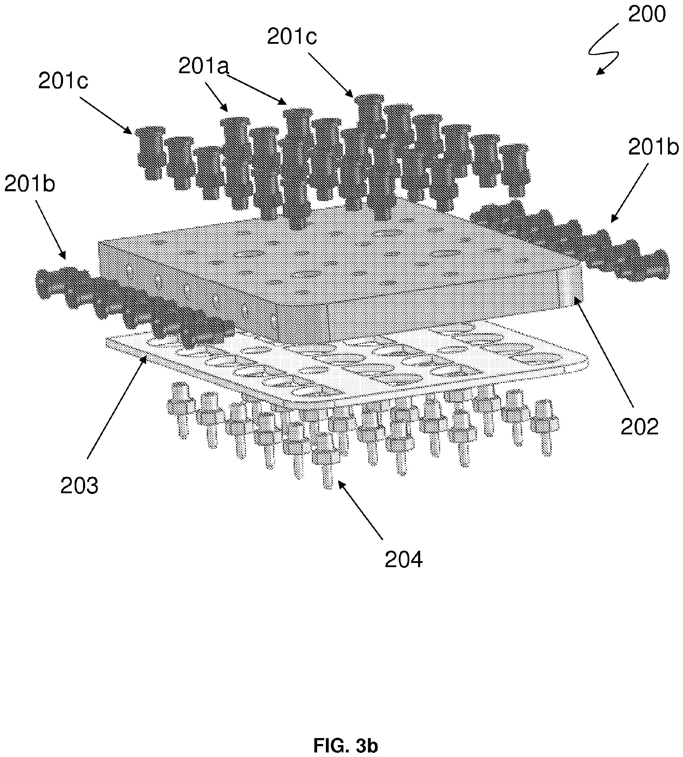

Un capot 200 avantageux, détaillé figure 3b, est préférentiellement constitué d'une plaque de Polycarbonate de 10 mm d'épaisseur dans laquelle 24 connecteurs de Polypropylène mâle 204 et 36 connecteurs de Polypropylène femelle 201 sont vissés (Ø4 mm 10-32 UNF). 24 de ces connecteurs femelle 201a et 201c sont situés sur le dessus du capot 200, et sont alignés avec les connecteurs male 201. Les douze autres 201b sont sur le flanc du capot 200. Ainsi, lorsque la boîte est refermée, l'étanchéité étant assurée par un joint plat de PDMS 203 de 2 mm d'épaisseur, il y a un connecteur mâle 204 et un ou deux connecteurs femelle 201 par puits 10 :
- pour les puits d'entrée 10a, il y a un seul connecteur de chaque type (201a et 204). L'entrée de fluide est sur le dessus du capot 200.
- Pour les puits de sortie 10b, on retrouve les deux connecteurs femelle 201b-c. Seul le connecteur latéral 201b sert au passage du fluide. Le connecteur femelle 201c constitue l'entrée du port 22.

Comme on le voit sur les figures 2a et 2b le connecteur femelle 201c et son connecteur mâle 204 sont alignés. C'est cela qui permet l'insertion de la pointe d'une pipette 3 de prélèvement à travers ces deux connecteurs à la fois, et donc à travers tout le capot 200.

### Circulation de fluide dans le boîtier inférieur - Système débulleur intégré

La figure 2b permet de visualiser le chemin fluidique dans un mode de réalisation préféré de la boite 1 qui comprend le capot 200 décrit précédemment. Le fluide pénètre par le connecteur femelle 201a. Il est transmis dans le puits d'entrée 10a par un connecteur mâle 204. Le fluide contenu dans ce puits d'entrée 10a est ensuite perfusé par une connectique 16 d'entrée de la chambre microstructurée 15, pour être recueilli dans le puits de sortie 10b via une connectique 16 de sortie de la chambre 15. L'excédent de fluide contenu dans le puits de sortie 10b est alors aspiré par un connecteur mâle 204 et acheminé vers le connecteur femelle 201b du capot. En prenant garde au volume utilisé, il est ainsi possible de perfuser en continu la chambre de culture. On rappelle que par « perfuser » un élément de la boite 1, que ce soit des puits 10 ou une chambre microstructurée 15, on entend le brancher fluidiquement, que ce soit pour l'alimenter en fluide, ou pour évacuer un trop plein de fluide. Les débits de circulation fluidiques sont en effet très faibles, et la communication fluidique dont il est question peut s'assimiler à du goutte à goutte à travers les différentes connectiques et connecteurs de la boite 1.

On remarque que le chemin fluidique fait un coude avant le connecteur femelle 201b. Cette architecture non limitative permet d'avoir les deux sorties indépendantes 201b et 201c pour un seul connecteur mâle 204. C'est une astuce de construction grâce à laquelle on peut avoir un port 22 de prélèvement spécifique, toujours disponible et apte à recevoir une micropipette 3, sans complexifier le circuit de fluide.

Dans ce cas, une bifurcation apparaît à l'intérieur du capot 200. Si l'on suit l'axe vertical du connecteur mâle 204 en sortant du puits 10b, on traverse le connecteur femelle 201c et donc on sort par le port de prélèvement 22, et si on prend la bifurcation, on sort sur le flanc du capot par le connecteur femelle 201b.

En outre, chaque puits 10 ayant un volume de l'ordre du millilitre (mL), les deux puits 10a-b par chambre 15 sont donc équivalent à un réservoir externe de 3mL. De plus, comme on le voit encore sur la figure 2b, tout point du puits 10a est situé avantageusement à une altitude supérieure à un point de la chambre. Par altitude supérieure, on sous-entend des conditions telles que toute goutte de fluide de la chambre 15 possède moins d'énergie potentielle de pesanteur que toute goutte du puits 10a. Cela est le cas en plaçant la boîte 1 selon l'invention dans une position de fonctionnement normale, c'est-à-dire avec le fond du boitier inférieur 100 posé sur une surface proche de l'horizontale.

Grâce à cette différence d'altitude, quand bien même une bulle d'air viendrait sortir du connecteur 204, la poussé d'Archimède la ramènerait en haut du puits 10a. Il serait contraire à la mécanique qu'elle puisse atteindre le fond du puits, et entrer dans la chambre 15. Comme il a déjà été dit, grâce à cette structure il est donc possible de débuller automatiquement le circuit à condition seulement de prendre garde au volume de fluide dans le puits 10a.

Cela est valable aussi lors du remplissage initial du circuit, lors duquel il fallait purger précautionneusement les tuyaux de toute bulle d'air pour les dispositifs d'art antérieur. Il y a une communication directe entre les puits 10 et la chambre 15 via la connectique 16. Une fois que cette dernière a été ensemencée et donc remplie par un opérateur, il suffit de remplir chaque puits 10 de liquide nutritif pour avoir l'assurance qu'il n'y a pas de bulle d'air enfermée dans le boitier inférieur 100, par simple gravité. Le capot 200 est refermé, et les bulles d'air éventuellement présentes dans le reste du circuit seront capturées par les puits 10a. Des tests ont été effectués, et il apparaît qu'un opérateur n'a besoin que de sept minutes pour mettre en perfusion après ensemencement une boîte multi-réacteurs complète, c'est-à-dire douze bioréacteurs. Cette opération prenait auparavant entre une heure et une heure trente, durant lesquelles l'opérateur devait injecter doucement du fluide dans chaque bioréacteur au moyen d'une seringue, remplir lentement le circuit de perfusion, puis le dégazer, ce qui n'a plus lieu d'être.

### Prélèvements élaborés

En plus des mécanismes de prélèvement décrits précédemment, on peut également envisager d'effectuer en parallèle des prélèvements en amont des chambres 15, notamment dans les puits d'entrée 10a, par exemple en y installant un autre port 22 similaire à celui des puits de sortie 10b. En comparant ces nouveaux prélèvements avec les prélèvements de sortie, notamment en soustrayant les grandeurs d'entrée aux grandeurs de sortie, on peut réaliser une mesure différentielle de la réaction cellulaire. Aucun système connu ne le permet, ceux-ci ne permettant que le prélèvement en un unique endroit qu'est le réservoir.

Dans un autre mode de réalisation encore plus avancé, on peut envisager de remplacer le prélèvement manuel à la pipette par une surveillance automatique via des sondes. En effet on dispose de sondes physico-chimiques miniaturisées, qui peuvent être introduites dans les ports de prélèvement 22 pendant tout ou partie de la culture cellulaire. La surveillance est alors continue.

Ces sondes sont généralement constituées d'un capteur, souvent piezorésistif, relié par un fil à un instrument de mesure, qui déduit une grandeur physique des données transmises par le capteur. On peut citer de façon non exhaustive les pH-mètres, les thermomètres, les conductimètres, les débitmètres, les manomètres, les néphélomètres ou encore les turbimètres, dont le capteur est placé dans au moins un puits 10b, le fil sortant par le port 22 colmaté par un matériau assurant l'étanchéité. La sonde peut en outre être l'extrémité d'une ou plusieurs fibres optiques, enregistrant le spectre d'émission et/ou d'absorbance du fluide à destination d'un spectrophotomètre, grâce auquel est calculée en permanence la concentration d'une ou plusieurs substances présentes en solution.

Avantageusement, on intègre la sonde dans le bouchon 205. Grâce à cela, l'étanchéité est directement assurée pendant toute la durée de fonctionnement de la sonde, et il reste possible d'ôter la sonde pour effectuer un prélèvement.

La possibilité d'une ou plusieurs sondes incorporées à la boîte selon l'invention n'est de plus pas incompatible avec la possibilité de mesures différentielles : on peut comparer une mesure automatique en entrée avec une mesure automatique en sortie.

On notera toutefois que l'invention n'est limitée à aucun de ces modes de réalisation.

### Structure du microsystème

Le microsystème 150 évoqué précédemment est une structure abritant éventuellement la ou les chambres microstructurées 15 et placée sous la plaque multi-puits 110. On remarquera que le microsystème 150 peut être composé de plusieurs éléments distincts, manipulables séparément. Chaque élément comprend alors une ou plusieurs des chambres microstructurées 15 que compte la boîte 1.

Ce microsystème 150 peut faire l'objet de nombreux modes de réalisation, mais s'adapte avantageusement à une plaque 24 puits telle que représentée sur les figures en étant constitué de douze chambres 15. Ces chambres 15, dont la surface est microstructurée, sont de dimensions identiques et disposées de façon à ce qu'il y en ait deux par rangée, chacune reprenant de façon particulièrement préférée mais sans y être limitée un bioréacteur suivant la demande de brevet FR0954288. L'invention n'est cependant par limitée à cette architecture, et il est seulement nécessaire d'avoir au moins un puits par chambre. Par exemple, dans un autre mode de réalisation, une boîte selon l'invention peut comprendre six chambres microstructurées et une plaque six puits.

Deux pièces visibles sur la figure 5 peuvent composer le microsystème 150 : une plaque inférieure 152 portant l'empreinte du fond des chambres 15, et une plaque supérieure 151.

Une fois ces deux plaques assemblées hermétiquement, les cavités qui apparaissent sont les chambres 15. Celles-ci sont des petits volumes dans lesquels les cellules vont se développer dans des conditions très favorables. En effet les microstructures forment des canaux complexes offrant une grande surface de contact avec le fluide.

La présence d'un microsystème 150 est toutefois optionnelle, d'autres structures pouvant abriter une chambre 15 de culture cellulaire étant connues de l'homme de l'art.

### Plaque supérieure du microsystème

Un matériau, le PolyDiMéthylSiloxane (PDMS), se prête tout particulièrement à la plaque supérieure 151, ainsi qu'au reste du microsystème 150. En effet, il est transparent, poreux à l'oxygène et aux gaz en général, et très adapté à la microstructuration, d'où son intérêt pour la culture cellulaire. En outre il est légèrement déformable.

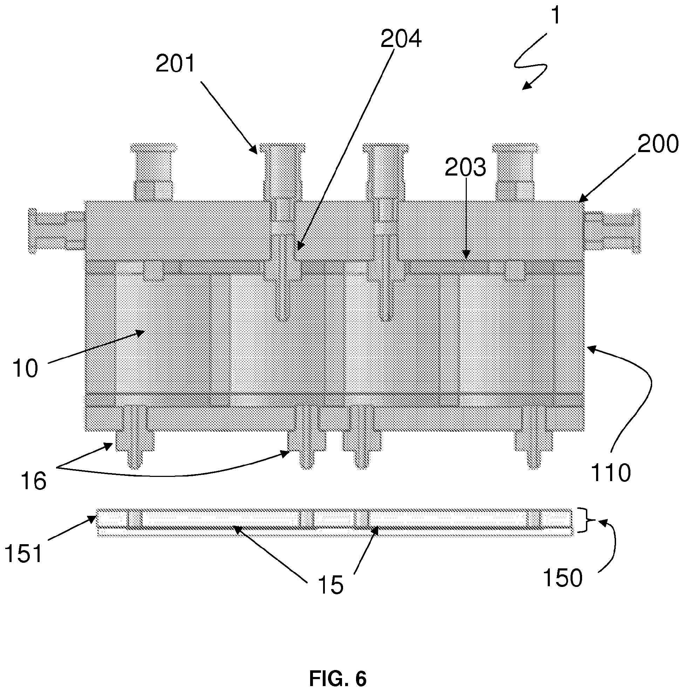

Dans le mode de réalisation représenté figure 6, la boîte 1 comprend un microsystème 150 et au moins une connectique 16 d'interface du microsystème 150, et au moins une chambre microstructurée 15 est branchée fluidiquement en entrée et/ou en sortie par une connectique 16 insérée de façon amovible dans un trou de la plaque supérieure 151.

En d'autres termes, la plaque supérieure 151 est pourvue de trous. Les connectiques 16 ne sont pas intégrantes au microsystème 150, mais sont solidaires du reste de la boîte 1. Les connectiques 16 sont alors préférentiellement des connecteurs en dur, fixés dans les trous 11 des puits 10, par exemple par vissage, mais encore par collage, moulage, ou toute autre technique connue. Ces connecteurs 16 permettent de perfuser la chambre microstructurée 15, mais s'insèrent de façon amovible dans les trous (ils sont par exemple enfoncés en force en utilisant l'élasticité du matériau, notamment siliconé, qui compose la plaque 151) pour pouvoir désolidariser facilement le microsystème 150 du reste de la boîte 1. Cette désolidarisation offre de nombreuses possibilités d'étude : marquages fluorescents, tests d'activité, détachement et comptage cellulaire... Le matériau des connectiques 16 est préférentiellement le polycarbonate ou le polypropylène.

La souplesse du PDMS est très appréciable pour la plaque 151. En jouant sur le diamètre des trous par élasticité, une jonction hermétique entre le microsystème et les connecteurs 16 est possible comme mentionné précédemment. Cela permet un fonctionnement sans risque de fuite, même après des désolidarisations et resolidarisations successives du microsystème 150.

### Observation au microscope des cellules pendant la culture

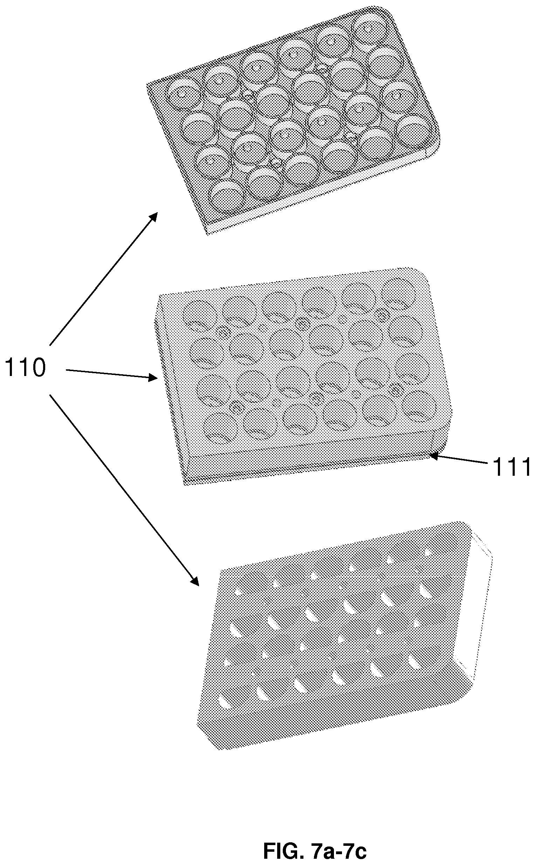

Grâce à leurs matériaux, le capot 200 et le microsystème 150 sont autoclavables : selon les besoins des utilisateurs, il est possible dans certains cas de les réutiliser pour une nouvelle culture après les avoir nettoyés et stérilisés. Pour la plaque multi-puits 110, de nombreux matériaux existent. Il est tout d'abord possible de choisir une plaque en Polystyrène, comme celle de la figure 7a. Une plaque 110 en ce matériau est à usage unique et doit être jetée en fin de culture. Elle est par contre peu chère et parfaitement transparente, rendant facile l'observation des cellules pendant la culture : l'encombrement de la boîte multi-réacteurs totale selon l'invention n'étant pas plus grand que celui d'une boîte de Pétri, on peut la placer en entier sous un microscope. Il est en effet souhaitable de pouvoir surveiller à tout moment les cellules sans les perturber, aussi bien pour s'assurer de la bonne croissance que pour observer l'impact toxicologique direct d'une substance.

Alternativement on peut choisir une plaque 110 en Polycarbonate, c'est-à-dire le même matériau que le capot 200. L'ensemble est alors autoclavable, mais le Polycarbonate possède l'inconvénient de devenir opaque lorsqu'il est usiné, ce qui est problématique pour l'observation des parties de chambre sous les puits. Une première solution, si l'on choisit une plaque multi-puits 110 en Polycarbonate comme celle représentée figure 7b, consiste à découper le fond des puits 10, et à interposer entre la boîte 110 et le microsystème 150 une fine plaque supplémentaire 111 de polycarbonate et un joint de PDMS assurant une étanchéité identique à celle entre le boitier 100 et le capot 200. Un moyen de serrage similaire au moyen 20 maintient alors le boitier inférieur uni. Grâce à cette plaque supplémentaire 111 parfaitement transparente car non usinée, l'observation au microscope est parfaite et en même temps l'ensemble est autoclavable.

Une seconde solution, plus astucieuse, consiste à choisir une plaque 110 avec des puits demi-lune, représentée figure 7c. En effet on remarque par exemple sur la figure 5 que la zone la plus intéressante d'observation est la partie centrale des chambres 15. En effet c'est là qu'est le coeur de la culture cellulaire, les parties latérales d'une chambre 15 étant plutôt des canaux de répartition de fluide. Et cette zone centrale ne se situe pas sous les puits 10, mais entre un puits 10a et un puits 10b, comme on le voit particulièrement sur la figure 2a ou 2b. Dans le cas d'une boîte demi-lunes, c'est une zone rectangulaire dégagée qui correspond au « plat » des puits demi-lune. Une plaque avec des puits demi-lune est donc parfaitement adaptée à l'observation, sans qu'il y ait besoin de modifier le fond des puits.

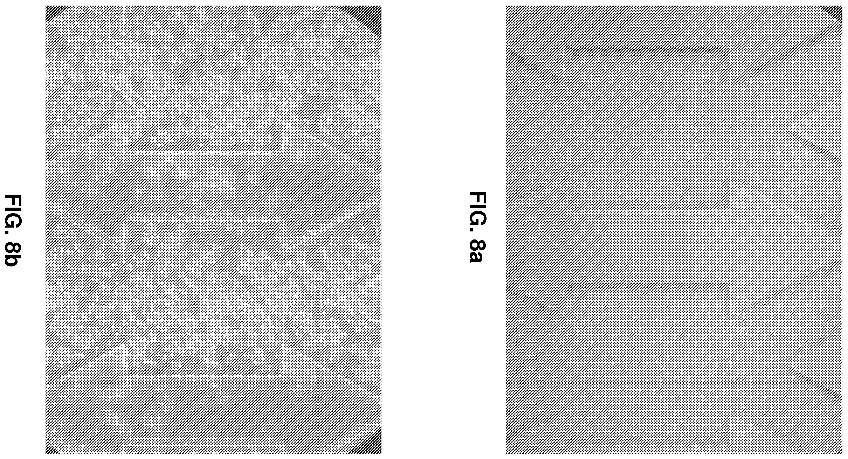

Les images obtenues en observant les cellules à travers la totalité de la boîte multi-bioréacteurs sont ainsi d'une précision remarquable, malgré l'épaisseur de plastique traversée. La figure 8a en est un exemple, une précision d'une dizaine de microns est atteinte, largement suffisante pour vérifier la présence des cellules, leur nombre, leur homogénéité, etc. Il est toutefois possible d'atteindre une précision optique encore supérieure dans le cas d'une boîte à microsystème 150 détachable (figure 6) décrite précédemment, ce qui permet notamment la vérification morphologique des cellules.

Pendant la culture cellulaire, il suffit ainsi d'arrêter la circulation de fluide, et de désolidariser les bioréacteurs du boîtier 100 en tirant doucement sur le microsystème 150 en PDMS. Il est envisageable, comme on l'a mentionné précédemment, d'avoir un microsystème 150 constitué de plusieurs éléments, chaque élément pouvant ne comprendre qu'une chambre 15. Ainsi, lorsqu'on veut observer une chambre 15 en particulier, on ne désolidarise que la partie du microsystème 150 qui la contient, et on peut laisser la circulation en marche pour le reste.

Le microsystème 150 (ou l'élément de microsystème) peut alors être introduit dans une enceinte stérile transparente et observé avec un niveau de détail et de contraste inégalé, puisque la vision est directe, à travers seulement quelques millimètres de plastique. On peut alors observer jusqu'à l'intérieur des cellules, comme le montre la figure 8b, pour par exemple analyser de façon très poussée certains effets d'une substance testée. Pendant la phase de désolidarisation, les puits 10 connectés au microsystème 150 sont vidés : la circulation étant arrêtée, le fluide peut être maintenu dans les fins canaux des connecteurs vissés 16 par simple capillarité. Le microsystème 150 est ensuite reconnecté au reste de la boîte 1 via les connecteurs 16, pour continuer et reprendre la perfusion. Il a été testé expérimentalement qu'il suffit de placer une goutte de milieu de culture dans chaque trou d'entrée et de sortie des chambres 15 avant de reconnecter l'ensemble pour éviter le risque d'introduction d'une bulle d'air dans une chambre 15.

### Système de culture cellulaire dynamique

Selon un autre aspect, l'invention concerne un système de culture cellulaire dynamique comprenant une boîte 1 selon le premier aspect de l'invention, au moins un circuit 300 de fluide et une pompe 320, préférentiellement une pompe péristaltique, ou toute autre moyen de circulation de fluide connu de l'homme de l'art. Les différents organes sont liés sur le circuit 300 par une tuyauterie 310 constituée de tubes, préférentiellement des tubes les plus courts possibles, et à la surface interne la moins adsorbante possible afin de limiter toute variation de concentration d'une substance injectée dans le circuit 300.

Le fait que la culture soit dynamique signifie qu'il y a recirculation en circuit fermé du fluide. Dans une telle configuration les conditions de développement des cellules sont plus proches de celles de l'organisme que dans les systèmes d'art antérieur dans lesquels le fluide soit est statique, soit n'effectue jamais une rotation complète de circuit (mouvement de va-et-vient de fluide dans une chambre, qui en particulier n'autorise pas de comparaison entre le fluide en entrée de chambre et celui sortant de la chambre, ou traversée unique de la chambre par un fluide qui n'est pas réutilisé).

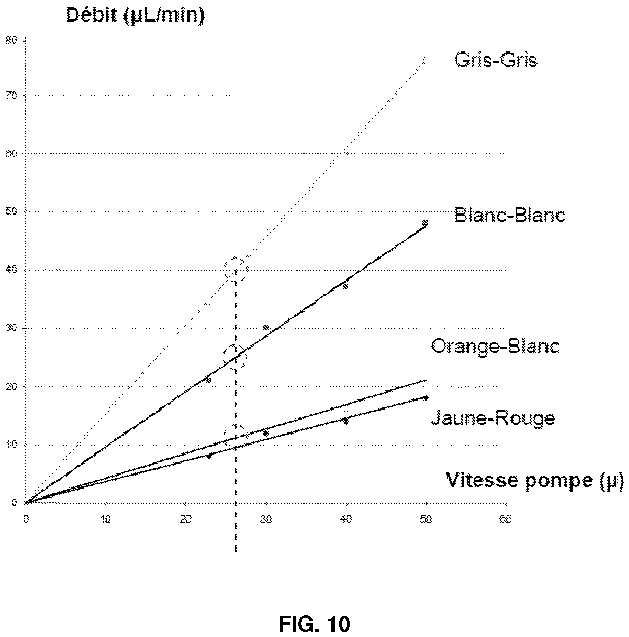

Une pompe péristaltique est une pompe dans laquelle le fluide est entraîné par compression et déformation d'un tube, à la manière des contractions musculaires autour de l'oesophage par exemple. Ce système est particulièrement adapté pour l'application à la culture cellulaire pour de nombreuses raisons. Tout d'abord, il s'applique principalement aux petits débits, pour lesquels il est d'une grande précision et d'une grande souplesse. L'arrêt de la pompe coïncide exactement avec l'immobilisation du fluide dans le circuit. Ensuite, il n'y a pas d'éléments autres que le tube flexible en contact avec le fluide. Il n'y a donc pas de risque de contamination ou de fuite. Enfin une seule pompe peut gérer plusieurs circuits de fluide à la fois, notamment 24 pour une pompe Ismatec® IPC-N particulièrement préférée. Cela correspond à la totalité des chambres de deux boîtes multi-réacteurs précédemment décrites. En jouant sur le diamètre interne des tuyaux 310, il est également possible de travailler en même temps avec des débits différents selon les circuits, ce qui peut par exemple être utile pour comparer différentes cinétiques d'exposition à une substance. Des tests ont par exemple montré qu'il était facilement possible d'avoir avec une seule pompe péristaltique des débits simultanés de 11, 25 et 40µL/min simultanément sur trois canaux en utilisant divers calibres de tuyaux standards, identifiées par un code couleur. La figure 10 illustre à cet effet par un graphique les débits obtenus en fonction de la vitesse de fonctionnement de la pompe pour quatre calibres de tuyaux. Le point de fonctionnement particulièrement préféré mentionné précédemment est placé sur le graphique.

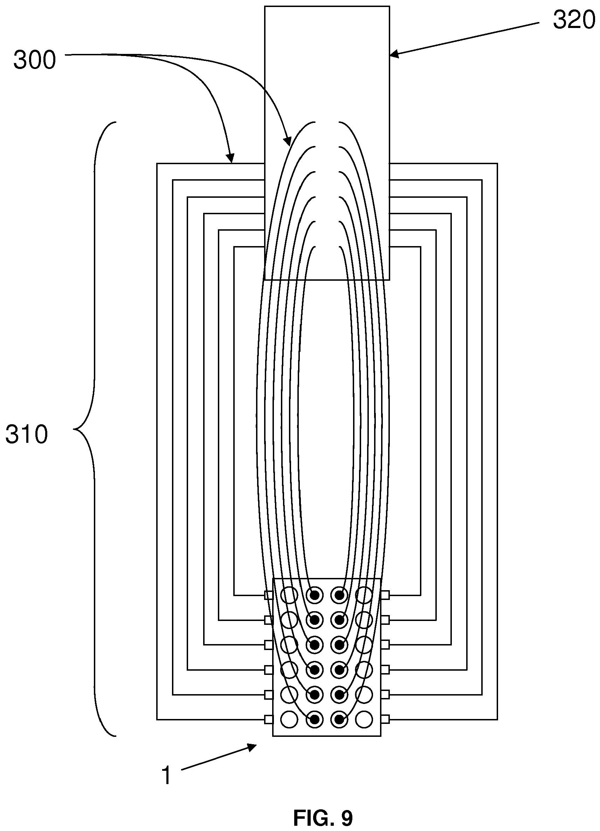

Un exemple de circuit avec une pompe 320 douze canaux et une boîte 1 selon l'invention est représenté figure 9. Il y a un circuit 300 de fluide par chambre 15. On remarque qu'aucun élément supplémentaire tel un réservoir ou un piège à bulle n'est requis. La longueur de chaque circuit 300 et donc les variations de concentrations des substances injectées, sont minimales. De plus, chaque circuit possède avantageusement son port 22 de prélèvement, et est donc testable indépendamment.

Dans cet exemple, toutes les cultures sont parallélisées. Grâce aux connectiques 201, il est toutefois possible de réaliser des architectures très variées, avec différents bioréacteurs en série ou en parallèle. Cet aspect modulaire possible grâce à l'aspect dynamique de la culture cellulaire du système selon l'invention permet de simuler des métabolismes complexes, par exemple un enchaînement de plusieurs organes bioartificiels et/ou de différents types de cellules caractéristiques d'un même organe. C'est ce que l'on appelle la co-culture. En effet la réponse métabolique à une substance pourrait être à plusieurs niveaux : ne pas affecter un premier organe mais affecter un second, entraîner la sécrétion d'une substance par un premier organe qui aurait des répercussions sur un second, etc. Dans un mode de réalisation particulièrement préféré, chaque chambre 15 de la boîte multi-réacteurs simule un organe différent (foie, rein, pancréas...), ces organes bioartificiels étant câblés selon de multiples circuits représentant l'architecture humaine au plus près (rein en aval du foie par exemple).

### Inserts à membranes

De façon encore plus avantageuse, la boîte selon l'invention permet de simuler certains « filtres » présents dans l'organisme. Ainsi, la barrière hémato-encéphalique est une barrière anatomique qui filtre et contrôle le passage des substances sanguines et les empêche de passer librement du sang au liquide extra-cellulaire du système nerveux central. Elle isole la substance grise du reste de l'organisme et lui permet d'avoir un milieu spécifique. Seules 2% des molécules la traversent librement. Des substances qui s'avéreraient ainsi neurotoxiques si injectées directement sans le cerveau pourrait s'avérer d'une totale innocuité si injectées normalement.

Pour prendre en considération cela, il est possible de placer dans les puits des inserts à membranes. Ces supports vendus dans le commerce se présentent sous la forme d'un petit disque présentant les propriétés physico-chimiques de la membrane anatomique à simuler, et s'installent dans les puits 10 d'entrée et de sortie de l'organe à isoler. La culture dynamique offerte par le système selon l'invention est encore une fois nécessaire pour utiliser cette possibilité. On peut ainsi par exemple compléter la structure décrite précédemment en simulant le passage dans le sang d'une molécule ingérée grâce à une muqueuse intestinale de synthèse.

### Méthode d'ensemencement cellulaire

Selon encore un autre aspect, l'invention concerne une méthode d'ensemencement cellulaire de la boîte multi-bioréacteurs. Comme il a été expliqué, les connectiques 16 peuvent recevoir la pointe d'une pipette 2.

Avant de pouvoir réaliser l'ensemencement à proprement parler, il est nécessaire d'accéder aux connectiques 16 en ouvrant le capot 200, en général après avoir déverrouillé les moyens de serrage 20. Dès lors, le fond des puits 10, et donc les connectiques 16, sont accessibles. L'opérateur utilise alors une pipette 2 comme instrument d'ensemencement.

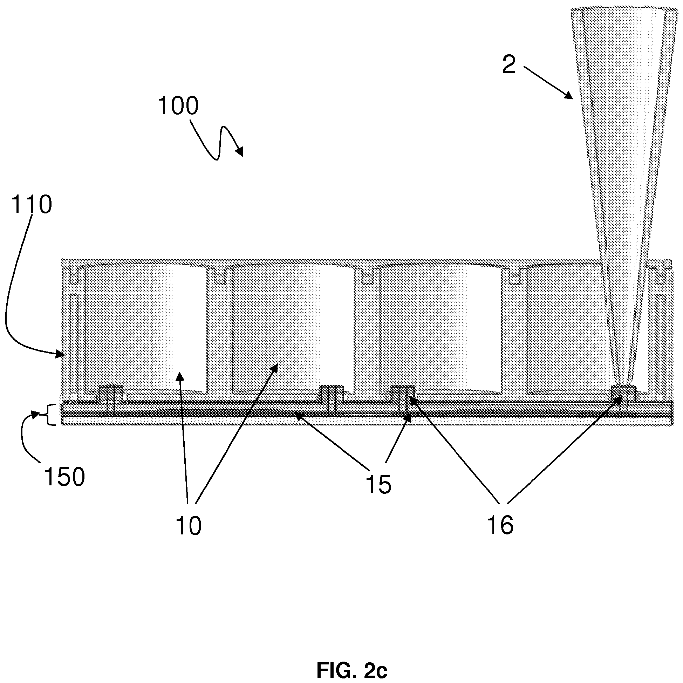

La configuration d'ensemencement est visible sur la figure 2c. Une fois chaque chambre ensemencée, il n'y a plus qu'à refermer le capot, connecter le ou les circuits 300, et lancer la perfusion par le fluide nutritif, pour initier le développement des cellules.

De façon préférée, la pipette 2 est une micropipette jaugée. Contrairement aux pipettes graduées, ces pipettes sont conçues pour contenir un volume particulier, pour lequel elles sont extrêmement précises. Puisque le volume d'une chambre 15 est connu, ainsi que la quantité de cellules à y injecter, il est possible de déterminer le volume optimal correspondant à l'ensemencement d'une chambre, et choisir une micropipette jaugée parfaitement adaptée.

Avec un tel instrument, l'opérateur n'a plus qu'à répéter pour chacune des chambres des étapes de : remplissage de la pipette 2, introduction de sa pointe dans une connectique 16 de la chambre, injection du contenu, et retrait de la pointe. Chaque ensemencement ne prend que quelques secondes, utilise une quantité rigoureusement connue et minimale de liquide cellulaire, et garantit des conditions de cultures optimales.

De façon particulièrement préférée, on utilisera une pipette multicanaux adaptée aux dimensions de la plaque multi-puits 110. Il s'agit d'une pipette comportant un seul manche, mais de multiples pointes (en l'occurrence six dans le cas d'une boîte 24 puits comme représentée dans les figures). Cet instrument permet d'ensemencer simultanément une chambre de chaque rangée, et donc d'ensemencer toute la boîte en seulement deux manipulations.

Une pipette multicanaux peut même être équipée sur un système de distribution automatisée. Un tel système comprend un bras robotisé et une alimentation ou un réservoir de fluide. Il se déplace tout seul d'une position à une autre afin d'ensemencer automatiquement toutes les chambres d'une boîte multi-bioréacteurs, voire de plusieurs. Ce genre de dispositif ouvre la voie à une culture de masse d'organes bioartificiels.

## Revendications

1. Boîte (1) comprenant un microsystème (150) et au moins une connectique (16) d'interface du microsystème (150); le microsystème (150) comprenant une plaque inférieure (152) portant l'empreinte d'au moins une chambre microstructurée de culture cellulaire dynamique (15), et une plaque supérieure (151), **caractérisée en ce que** l'au moins une chambre microstructurée (15) est branchée fluidiquement en entrée et/ou en sortie par l'au moins une connectique (16) insérée de façon amovible dans un trou de la plaque supérieure (151), la boîte (1) comprennant en outre au moins un puits (10), la chambre microstructurée (15) étant connectée à un puits (10) via une des connectiques (16).

2. Boîte selon la revendication précédente, **caractérisée en ce que** le fond du puits (10) est à une altitude supérieure à tout point de la chambre (15).

3. Boîte selon la revendication précédente, **caractérisée en ce que** la connectique (16) est un connecteur fixé à un trou (11) au fond du puits (10).

4. Boîte selon l'une des revendications 1 à 3, **caractérisée en ce que** chaque chambre microstructurée (15) est connectée à un puits d'entrée (10a) et un puits de sortie (10b) perfusés chacun par une connectique (16) respectivement d'entrée et de sortie.

5. Boîte selon la revendication précédente, **caractérisée en ce qu'**elle est constituée d'un boitier inférieur (100) recouvert d'un capot étanche (200) maintenu par des moyens de serrage (20).

6. Boîte selon la revendication précédente, **caractérisée en ce que** le boitier inférieur (100) comprend le microsystème (150) et une plaque multi-puits (110), les puits (10) étant des puits de ladite plaque multi-puits (110).

7. Boîte selon la revendication précédente, **caractérisée en ce que** la plaque multi-puits (110) est une plaque jetable en polystyrène.

8. Boîte selon la revendication 6, **caractérisée en ce que** la plaque multi-puits (110) est une plaque autoclavable en polycarbonate.

9. Boîte selon l'une des revendications 5 à 8, **caractérisée** en ce le capot comprend des connectiques mâle (204) perfusant les puits (10) et des connectiques femelle (201) d'entrée.

10. Boîte selon la revendication précédente, **caractérisée en ce qu'**elle comprend un connecteur male (204) et un connecteur femelle (201a) par puits d'entrée (10a) et un connecteur maie (204) et deux connecteurs femelle (201b-c) par puits de sortie (10b).

11. Boîte selon l'une des revendications précédentes, **caractérisée en ce que** le microsystème (150) est constitué de polydimethylsiloxane.

12. Boîte selon l'une des revendications précédentes, **caractérisée en ce qu'**un insert à membrane est disposé dans au moins l'un des puits (10).

13. Système de culture cellulaire dynamique **caractérisé en ce qu'**il comprend
- une boîte (1) selon l'une des revendications précédentes, et
- au moins un circuit (300) de fluide comprenant une tuyauterie (310) de circulation munie d'un moyen (320) de circulation et connectée à au moins une chambre microstructurée (15).

14. Système selon la revendication précédente **caractérisé en ce que** le moyen (320) de circulation est une pompe péristaltique multicanaux.

## Patentansprüche

1. Einheit (1), umfassend ein Mikrosystem (150) und mindestens eine Schnittstellenanschlusstechnik (16) des Mikrosystems (150); wobei das Mikrosystem (150) eine untere Platte (152), die den Abdruck mindestens einer mikrostrukturierten Kammer einer dynamischen Zellkultur (15) trägt, und eine obere Platte (151) umfasst, **dadurch gekennzeichnet, dass** mindestens eine mikrostrukturierte Kammer (15) am Einlass und/oder am Auslass mittels mindestens einer Anschlusstechnik (16) fluidisch gekoppelt ist, die lösbar in ein Loch der oberen Platte (151) eingesetzt ist, wobei die Einheit ferner mindestens eine Vertiefung (10) umfasst, wobei die mikrostrukturierte Kammer (15) über eine der Anschlusstechniken (16) mit einer Vertiefung (10) gekoppelt ist.

2. Einheit nach vorangehendem Anspruch, **dadurch gekennzeichnet, dass** der Boden der Vertiefung (10) in einer gegenüber jedem Punkt der Kammer (15) höheren Höhe ist.

3. Einheit nach vorangehendem Anspruch, **dadurch gekennzeichnet, dass** die Anschlusstechnik (16) ein Verbinder ist, der in einem Loch (11) auf dem Boden der Vertiefung (10) befestigt ist.

4. Einheit nach einem der Ansprüche 1 bis 3, **dadurch gekennzeichnet, dass** jede mikrostrukturierte Kammer (15) an eine Einlassvertiefung (10a) und an eine Auslassvertiefung (10b) gekoppelt ist, die jeweils mit einer jeweiligen Einlass- und Auslass-Anschlusstechnik (16) perfundiert sind.

5. Einheit nach vorangehendem Anspruch, **dadurch gekennzeichnet, dass** sie von einem unteren Gehäuse (100) gebildet ist, das mit einer dichten Abdeckung (200) bedeckt ist, die von Spannmitteln (20) gehalten wird.

6. Einheit nach vorangehendem Anspruch, **dadurch gekennzeichnet, dass** das untere Gehäuse (100) das Mikrosystem (150) und eine Multivertiefungsplatte (110) umfasst, wobei die Vertiefungen (10) Vertiefungen der Multivertiefungsplatte (110) sind.

7. Einheit nach vorangehendem Anspruch, **dadurch gekennzeichnet, dass** die Multivertiefungsplatte (110) eine Einwegplatte aus Polystyrol ist.

8. Einheit nach Anspruch 6, **dadurch gekennzeichnet, dass** die Multivertiefungsplatte (110) eine im Autoklav behandelbare Platte aus Polycarbonat ist.

9. Einheit nach einem der Ansprüche 5 bis 8, **dadurch gekennzeichnet, dass** die Abdeckung männliche Anschlusstechnik (204) umfasst, die die Vertiefungen (10) perfundiert, und weibliche Einlass-Anschlusstechnik (201).

10. Einheit nach vorangehendem Anspruch, **dadurch gekennzeichnet, dass** sie einen männlichen Verbinder (204) und einen weiblichen Verbinder (201a) je Einlassvertiefung (10a) und einen männlichen Verbinder (204) und zwei weibliche Verbinder (201b-c) je Auslassvertiefung (10b) umfasst.

11. Einheit nach einem der vorangehenden Ansprüche, **dadurch gekennzeichnet, dass** das Mikrosystem (150) von Polydimethylsiloxan gebildet ist.

12. Einheit nach einem der vorangehenden Ansprüche, **dadurch gekennzeichnet, dass** ein Einsatz mit Membran in mindestens einer der Vertiefungen (10) angeordnet ist.

13. System für dynamische Zellkultur, **dadurch gekennzeichnet, dass** es umfasst
- eine Einheit (1) nach einem der vorangehenden Ansprüche, und
- mindestens einen Fluidkreis (300), der eine Zirkulationsrohrleitung (310) umfasst, die mit einem Zirkulationsmittel (320) ausgestattet ist und an mindestens eine mikrostrukturierte Kammer (15) gekoppelt ist.

14. System nach vorangehendem Anspruch, **dadurch gekennzeichnet, dass** das Zirkulationsmittel (320) eine peristaltische Multikanalpumpe ist.

## Claims

1. A box (1) comprising a microsystem (150) and at least one set of interface connectors (16) of the microsystem (150); the microsystem (150) comprising a lower plate (152) bearing the impression of at least one microstructured chamber of dynamic cellular culture (15), and an upper plate (151), **characterized in that** the at least one microstructured chamber (15) is connected fluidically at inlet and/or at outlet by the at least one set of connectors (16) inserted detachably into a hole of the upper plate (151), the box further comprising at least one well (10), the microstructured chamber (15) being connected to a well (10) via one of the sets of connectors (16).

2. The box according to the preceding claim, **characterized in that** the bottom of the well (10) is at an altitude higher than any point of the chamber (15).

3. The box according to the preceding claim, **characterized in that** the set of connectors (16) is a connector fixed to a hole (11) at the bottom of the well (10).

4. The box according to one of claims 1 to 3, **characterized in that** each microstructured chamber (15) is connected to an inlet well (10a) and an outlet well (10b) each perfused by a set of connectors (16) respectively inlet and outlet.

5. The box according to the preceding claim, **characterized in that** it consists of a lower casing (100) covered by a sealed hood (200) held by clamping means (20).

6. The box according to the preceding claim, **characterized in that** the lower casing (100) comprises the microsystem (150) and a multi-well plate (110), the wells (10) being wells of said multi-well plate (110).

7. The box according to the preceding claim, **characterized in that** the multi-well plate (110) is a disposable plate made of polystyrene.

8. The box according to claim 6, **characterized in that** the multi-well plate (110) is an autoclavable plate made of polycarbonate.

9. The box according to one of claims 5 to 8, **characterized in that** the hood comprises sets of male connectors (204) perfusing the wells (10) and sets of female inlet connectors (201).

10. The box according to the preceding claim, **characterized in that** it comprises a male connector (204) and a female connector (201a) per inlet well (10a) and a male connector (204) and two female connectors (201b-c) per outlet well (10b).

11. The box according to one of the preceding claims, **characterized in that** the microsystem (150) consists of polydimethylsiloxane.

12. The box according to one of the preceding claims, **characterized in that** a membrane insert is arranged in at least one of the wells (10).

13. A dynamic cellular culture system **characterized in that** it comprises
- a box (1) according to one of the preceding claims, and
- at least one fluid circuit (300) comprising circulation piping (310) fitted with circulation means (320) and connected to at least one microstructured chamber (15).

14. The system according to the preceding claim, **characterized in that** the circulation means (320) is a multichannel peristaltic pump.
